# EUROPEAN PATENT APPLICATION

(11) **EP 0 773 040 A2**
(43) Date of publication of application: **14.05.1997**
(21) Application number: 96830538.3
(22) Date of filing: 21.10.1996
(51) Int. Cl.: A61N 2/06

(54) **Therapeutic effect eyeglass frame**

(30) Priority: 10.11.1995 IT MI950776 U
(71) Applicant: Filospiave Group S.p.A., 31040 Segusino, Treviso (IT)
(72) Inventor: Lozza, Lucio, c/o Filospiave Group S.p.A., 31040 - Segusino, Treviso (IT)
(74) Representative: Cicogna, Franco

(57) **Abstract**

The present invention relates to a therapeutic effect eyeglass construction which comprises at least a permanent magnet, made of neodymium magnetized metal elements, which is associated with the eyeglass frame.

## Description

### BACKGROUND OF THE INVENTION

The present invention relates to a therapeutic effect eyeglass construction.

As is known, magnetotherapy is a therapy which is based on the use of magnetic fields affecting the human body, so as to provide several magnetic effects.

At present the magnetotherapy applications are performed by apparatus specifically designed for affecting the human body by comparatively high magnetic fields which are conventionally generated by annular movable elements practically encompassing a patient body.

Since the magnetic field generators are usually spaced away from the human body, they must have a comparatively high magnetic power thereby the generating apparatus are very expensive.

### SUMMARY OF THE INVENTION

Accordingly, the aim of the present invention is to allow the possibility of performing a magnetic therapy by using small size magnetic elements adapted to provide a continuous magnetic action.

Within the scope of the above mentioned aim, a main object of the present invention is to provide such an eyeglass frame affording very good therapeutic effects while being very simple from a mere construction standpoint.

Another object of the present invention is to provide such an eyeglass construction which is very reliable and safe in operation.

Yet another object of the present invention is to provide such an eyeglass construction which can be easily made starting from easily commercially available elements and materials and which, moreover, is very competitive from a mere economic standpoint.

According to one aspect of the present invention, the above mentioned aim and objects, as well as yet other objects, which will become more apparent hereinafter, are achieved by a therapeutic effect eyeglass construction, characterized in that said eyeglass construction comprises at least a permanent magnet associated with the eyeglass frame.

### BRIEF DESCRIPTION OF THE DRAWINGS

Further characteristics and advantages of the present invention will become more apparent hereinafter, from the following detailed disclosure of a therapeutic effect eyeglass construction according to the invention which is illustrated, by way of an indicative, but not limitative, example in the accompanying drawing, where:
Figure 1 is a front view of an eyeglass construction according to the present invention; and
Figure 2 is a side view of the eyeglass construction according to the present invention.

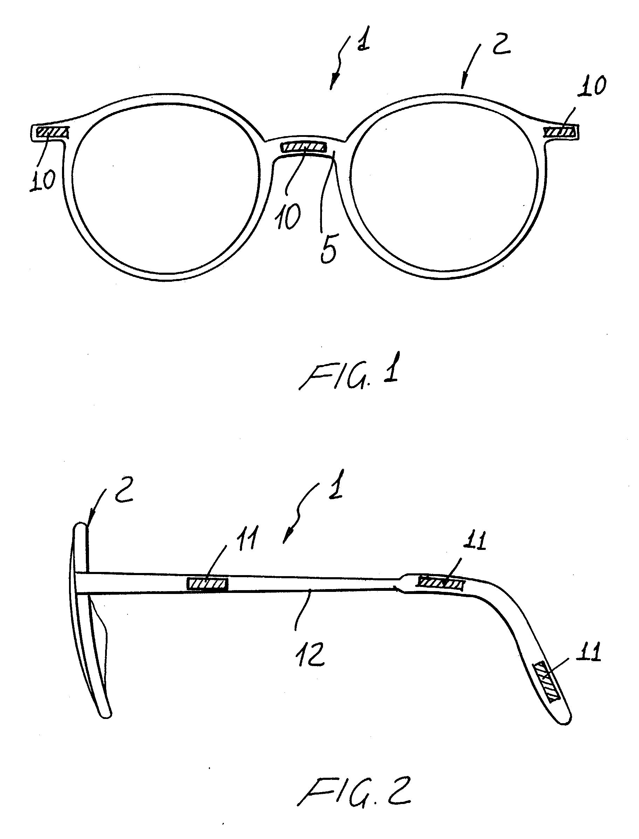

### DESCRIPTION OF THE PREFERRED EMBODIMENTS

With reference to the above mentioned figures, the therapeutic effect eyeglass construction according to the present invention, which has been generally indicated by the reference number 1, comprises an eyeglass frame 2, which can have any suitable desired shape.

The main feature of the present invention is that at least a permanent magnetic, made of neodymium magnetized metal elements is associated with said eyeglass frame.

Said permanent magnet can be either applied on the outside of the eyeglass frame, or it can be embedded in said eyeglass frame.

According to a preferred embodiment of the invention, the permanent magnet, indicated by the reference number 10, is embedded in the bridge element 5 of the eyeglass frame 2 and, thus, it will provide its magnetic effect at the front region of the head of the user.

According to a further embodiment of the invention, the permanent magnet, indicated by the reference number 11, is directly embedded in the eyeglass arms 12, at any suitable positions.

Thus, in the latter embodiment, the permanent magnet will be arranged on a side of the head, at the temple region, and will provide a continuous action adapted to enhance the typical magnetic action of the human body.

Thus, the provision of the permanent magnet directly in the eyeglass frame will provide a continuous contact with the user body to provide a constant magnetic effect exerted near the user body.

In practicing the invention, the used materials, provided that they are compatible to the intended application, as well as the contingent size and shapes, can be any depending on requirements.

## Claims

1. A therapeutic effect eyeglass construction, characterized in that said eyeglass construction comprises at least a permanent magnet associated with the eyeglass frame.

2. A therapeutic effect eyeglass construction, according to Claim 1, characterized in that said permanent magnet comprises neodymium magnetized metal elements.

3. A therapeutic effect eyeglass construction, according to Claim 1, characterized in that said permanent magnet is embedded in said eyeglass frame.

4. A therapeutic effect eyeglass construction, according to Claim 1, characterized in that said permanent magnet is arranged at a bridge element of said eyeglass frame.

5. A therapeutic effect eyeglass construction, according to Claim 1, characterized in that said permanent magnet is arranged at arm members of said eyeglass construction.
